# FASCICULE DE BREVET EUROPEEN

(11) **EP 1 204 865 B1**
(45) Date de publication et mention de la délivrance du brevet: **22.11.2006**
(21) Numéro de dépôt: 00958689.2
(22) Date de dépôt: 14.08.2000
(51) Int. Cl.: G01N 33/531, G01N 33/68

(54) **COMPOSES SYNTHETIQUES PORTANT DEUX EPITOPES POUR IMMUNODOSAGES**
SYNTHETISCHE VERBINDUNGEN MIT ZWEI EPITOPEN FÜR IMMUNOASSAYS
USE OF STABILISED SYNTHETIC COMPOUNDS IN IMMUNOASSAY

(30) Priorité: 16.08.1999 FR 9910526
(43) Date de publication de la demande: 15.05.2002
(73) Titulaire: Bio-Rad Pasteur, 92430 Marnes-la-Coquette (FR)
(72) Inventeur: GARRIC, Isabelle, Nathalie, F-78330 Fontenay-le-Fleury (FR); GIULIANI, Isabelle, Karine, F-69780 Mions (FR); LARUE, Catherine, Christiane, Marie, F-92420 Vaucresson (FR); RIEUNIER, François, Yves, F-78390 Bois d'Arcy (FR); TRINQUIER, Sylvie, Marie-France, F-67000 Strasbourg (FR)
(74) Mandataire: Bernasconi, Jean Raymond
(86) Numéro de dépôt international: PCT/FR2000/002323
(87) Numéro de publication internationale: WO 2001/013114

(56) Documents cités:
- EP-A- 0 650 053
- EP-A- 0 752 426
- WO-A-92/06998
- WO-A-96/27661
- WO-A-98/24816
- US-A- 5 106 834

## Description

La présente invention concerne des composés synthétiques utilisables comme étalons ou témoins dans les immunoessais, notamment pour le dosage de la troponine I, leur procédé de préparation, des compositions et des trousses contenant de tels composés ainsi que les procédés d'immunodosage mettant en oeuvre de tels composés.

On sait que la troponine est un complexe protéique myofibrillaire, constitué de trois protéines, les troponines I, T et C. Ce complexe protéique permet de contribuer à la régulation de la contraction du muscle par l'ion Ca²⁺, en interagissant avec la myosine et l'actine. De façon plus précise, on sait que lorsqu'un influx nerveux arrive au niveau de la plaque motrice d'un muscle, il y a génération d'un potentiel d'action qui est transmis au réticulum sarcoplasmique. Le Ca²⁺ est alors libéré dans le cytosol et se fixe sur la troponine C, ce qui entraîne un renforcement de l'interaction entre la troponine l et la troponine C et, par suite, un changement de conformation du complexe troponine l, T, C. Il y a alors libération des sites d'interaction actine-myosine, ce qui permet le mouvement de contraction du muscle.

Lorsque le muscle est endommagé, que ce soit le muscle cardiaque, lors d'une nécrose myocardique consécutive à un infarctus du myocarde, ou que ce soit le muscle squelettique lors d'efforts physiques prolongés, les troponines alors libérées apparaissent plus ou moins rapidement dans la circulation sanguine.

Ainsi on a récemment préconisé le dosage de la troponine pour le diagnostic précoce de l'infarctus du myocarde, que ce soit celui de la troponine T dans *Circulation (1991,) 83*, *pp. 902-912,* ou de la troponine I *dans Am. Heart J. (1987), 110, pp. 1333-1344,* et *Molecular Immunology (1992),* *29* (*2*), *pp. 271-278.* De même, on a proposé le dosage de la troponine T cardiaque pour mesurer le succès de la thérapie thrombolytique suite à un infarctus du myocarde dans *Br. Heart J., (1994), 71, pp. 242-248,* ainsi que le dosage de la troponine I squelettique pour la mesure du dommage des muscles squelettiques (abrégé n° 35 de *l'American Association for Clinical Chemistry, 46th National Meeting, New Orleans, July 17-21, 1994*). Il est à noter que le dosage des différentes troponines cardiaques et squelettiques est aujourd'hui un moyen très utile pour le diagnostic de pathologies humaines et animales.

Il est bien connu que les immunoessais pratiqués dans les laboratoires d'analyses biologiques nécessitent la fourniture par le fabricant, à côté des réactifs nécessaires au dosage (c'est-à-dire des anticorps, marqués ou non, des agents de révélation et des solutions de dilution), d'un étalon du composé à déterminer qui, mis en oeuvre dans des conditions analogues à celles de l'échantillon à étudier, servira de référence pour le calcul des résultats et/ou de témoin positif.

Pour obtenir l'étalon et/ou le témoin du composé à déterminer, on peut utiliser ledit composé purifié sous forme lyophilisée (accompagné d'un solvant dans lequel le composé sera dissous par l'utilisateur avant l'emploi) ou prêt à l'emploi.

Du fait que les réactifs biologiques sont instables, les solutions étalons ou témoins préparées à partir de lyophilisat sont congelées en doses unitaires et conservées à -80°C. On a constaté par ailleurs que ces solutions n'étaient pas stables plus de quelques heures à +4°C, même si des inhibiteurs de protéases ou des agents antibactériens y étaient ajoutés. Ceci oblige donc les utilisateurs à préparer extemporanément leurs solutions d'étalonnage.

La demande de brevet publiée sous le numéro FR-A- 2 701 954, divulgue une composition stabilisée de troponine I ou T pour immunoessai caractérisée en ce qu'elle est constituée d'une solution aqueuse contenant de la troponine I ou de la troponine T, en mélange avec de la troponine C et notamment dans des proportions de 1 à 10 équivalents molaires de troponine C par équivalent de troponine I ou T, et du chlorure de calcium. Cette technique permet la conservation durant plusieurs jours à +4°C des solutions étalons, plus ou moins diluées, de troponine I ou T.

La demande de brevet publiée sous le numéro FR 2 734 267 décrit des solutions étalons de la troponine composées d'un complexe ternaire formé par la troponine I, la troponine T et la troponine C.

Les matières premières utilisées pour obtenir ces étalons sont d'origine humaine ou animale, et les étalons ou témoins ainsi obtenus sont stables pendant environ un mois à +4°C.

La demande WO 94/15217 décrit certains peptides synthétiques utiles comme immunogènes pour la préparation des anticorps reconnaissant le peptide N-terminal de la troponine I. Certains de ces peptides peuvent être utilisés comme étalons dans des immunodosages de la troponine I, mettant en oeuvre les anticorps objets de l'invention couverte par la demande WO 94/15217.

De même, la demande de brevet WO 94/27156 porte sur un procédé de dosage de la troponine I cardiaque, mettant en oeuvre des anticorps spécifiques de la troponine I cardiaque. Ces anticorps peuvent être préparés à partir de fragments peptidiques ayant une séquence absente de la troponine I du muscle squelettique et donc spécifique de la troponine I cardiaque. Toutefois, cette demande ne divulgue ni ne suggère la possibilité d'utiliser certains fragments peptidiques comme étalons dans les immunodosages de la troponine I.

On connaît également de la demande WO 96/27661 des solutions aqueuses pour stabiliser des protéines et des peptides. Ces solutions trouvent notamment leur application dans les tests diagnostiques de protéines ou peptides.

Selon WO 97/27661, de telles solutions aqueuses permettent même d'augmenter la stabilité des fragments de la troponine I qui sont connus pour être moins stables que la troponine I entière.

La demande EP-A-752 426 publiée le 8 janvier 1997 décrit aussi des étalons de la troponine I composés d'un ou de plusieurs peptides fixés sur une molécule porteuse telles que des protéines de haut poids moléculaire (>100 KD) ou des polymères.

La demande EP-A-650 053 décrit des étalons synthétiques contenant des sites actifs pour un ou plusieurs récepteurs, reliés entre eux par une structure arborescente. Cette demande décrit plus spécialement des étalons synthétiques de la troponine T qui ne sont stables en solution que pendant 3 semaines.

WO 98/24816 décrit des composés synthétiques biépitopiques utilisables comme étalons dans les dosages biologiques de la troponine I.

Ces composés sont constitués de structures linéaires comprenant deux séquences peptidiques épitopiques différentes de la troponine I reliées entre elles par un groupe de liaison ("linker') formé d'un squelette hydrocarboné et/ou d'un squelette peptidique, dans lequel les deux épitopes peuvent en outre être liés respectivement par leurs extrémités N- et C-terminales à des séquences peptidiques additionnelles.

Les composés synthétiques épitopiques ainsi obtenus présentent une excellente stabilité en solution, mais une insuffisante linéarité de dilution.

Par "linéarité de dilution", on entend la fonction représentant le signal obtenu en fonction du facteur de dilution du composé de référence, laquelle fonction doit idéalement être représentée par une droite.

On connaît, par ailleurs, avec la demande WO 95/04543 des structures synthétiques peptidiques constituées par une matrice de nature peptidique comprenant des ramifications formées par des peptides branchés sur les groupements latéraux du peptide matrice par l'intermédiaire d'une liaison dendritique.

Ces structures peptidiques ramifiées sont utiles pour la conception de protéines présentant une topologie hélicoïdale déterminée.

Les travaux des inventeurs ayant abouti à la présente invention ont permis de découvrir que des structures ramifiées du type décrit dans WO 95/04543 comprenant des bras latéraux branchés portant au moins deux séquences épitopiques différentes de la troponine I fournissaient des étalons ou témoins pour des immunoessais en vue du dosage de la troponine I présentant à la fois une stabilité élevée en solution à 4°C et une excellente linéarité de dilution.

L'invention a pour objet l'utilisation d'au moins un composé synthétique comprenant :
- une molécule porteuse possédant une chaîne hydrocarbonée peptidique formée
   a) par des enchaînements de 7 à 50 acides aminés formant une chaîne hydrocarbonée ouverte comprenant au moins un enchaînement introduisant une rigidité dans la molécule de sorte que les enchaînements formés par les atomes de la chaîne hydrocarbonée ne soient plus tous en libre rotation ; ou
   b) par des enchaînements de 8 à 50 acides aminés formant un cycle ; ladite chaîne hydrocarbonée comprenant au moins deux résidus sélectionnés dans le groupe constitué des acides aminés à chaînes latérales basiques, des acides aminés à chaînes latérales chargées négativement, des acides aminés à chaînes latérales hydroxylées et des acides aminés à chaînes latérales contenant du soufre, permettant le greffage de bras latéraux sur ladite chaîne hydrocarbonée; et
   et
- au moins deux épitopes différents portés par lesdits bras latéraux greffés sur ladite molécule porteuse,
comme étalon ou témoin pour des immuno-dosages de molécules biologiques, notamment de polypeptides ou protéines.

Il est souhaitable d'avoir un espacement minimum des fonctions réactives, ou susceptibles de réagir, sur lesquelles sont greffés les bras latéraux portant des épitopes.

A cet égard, on préfère que les épitopes soient espacés sur les bras latéraux d'au moins 40 Å, ce qui correspond à un espacement d'au moins trois acides aminés.

On préfère en outre que les bras latéraux qui portent les épitopes soient disposés sur la molécule porteuse de manière à ce qu'il n'existe pas de gène stérique ni d'interaction entre lesdits bras, en particulier lors de la réaction immunologique d'un ou plusieurs de ces épitopes avec des anticorps.

Avantageusement, les bras latéraux sont disposés de manière à former entre eux un angle, celui-ci pouvant être obtenu par l'introduction d'une torsion ou rigidité dans la chaîne hydrocarbonée de manière à ce que les bras latéraux soient disposés dans des orientations non parallèles, et, de préférence, opposées.

La chaîne hydrocarbonée est avantageusement un oligopeptide ou polypeptide formé d'acides aminés naturels, tels que ceux trouvés dans les protéines procaryotes ou eucaryotes, ou encore d'acides aminés non naturels.

On préfère dans ce cas que deux bras latéraux consécutifs soient portés par deux acides aminés tels que définis précédemment espacés l'un de l'autre par un nombre 2n+1 d'acides aminés, de préférence ne comportant pas de chaîne latérale réactive ou comportant une chaîne latérale réactive inactivée, où n représente un nombre entier et est avantageusement compris entre 1 et 6. De préférence n = 2, les valeurs n = 3, n = 4 et n = 5 donnant toutefois de bons résultats.

Selon un premier mode de réalisation de l'invention, la chaîne hydrocarbonée est ouverte et comprend au moins un enchaînement introduisant une rigidité dans la molécule de sorte que les enchaînements formés par les atomes de la chaîne hydrocarbonée ne soient plus tous en libre rotation.

Il peut par exemple s'agir d'un atome de carbone sp², par exemple lorsque la chaîne hydrocarbonée n'est pas, ou pas exclusivement, de nature peptidique, ou d'un atome de carbone d'un cycle

Lorsque la chaîne hydrocarbonée est de nature peptidique, l'écartement entre deux bras latéraux consécutifs permettant d'éviter les interactions entre les atomes d'un latéral avec les atomes de l'autre bras latéral est obtenu en prévoyant un espacement de 2n + 1 acides aminés entre les acides aminés portant les bras latéraux, n étant défini ci-dessus, ou en introduisant dans la chaîne peptidique un acide aminé cyclique générant une rigidité dans cette chaîne, ou encore en prévoyant simultanément l'une et l'autre de ces alternatives.

La rigidité de la molécule porteuse peut être, par exemple, conférée par un résidu de proline.

Selon un deuxième mode de réalisation de l'invention, la chaîne hydrocarbonée, notamment oligopeptidique ou polypeptidique, est fermée de manière à former un cycle.

Une séquence peptidique permettant de satisfaire au mieux les conditions mentionnées ci-dessus pour obtenir l'écartement souhaité répond à la formule:

X₁-A₁-A₂-A₃-A₄-A₅-X₂-A₆

dans laquelle
X₁ et X₂, identiques ou différents, sont les acides aminés portant les bras latéraux et sont choisis parmi un acide aminé à chaîne latérale basique, un acide aminé à chaîne latérale chargée négativement, un acide aminé à chaîne latérale hydoxylée, et un acide aminé à chaîne latérale soufrée, X₁ et X₂ de représentant de préférence la lysine.
A₁ et A₂ représentent un acide aminé choisi parmi la proline, la phénylalanine, la glycine, l'alanine, la valine, la leucine et l'isoleucine;
   sous réserve que l'un de A₁ et A₂ soit choisi parmi la proline et la phénylalanine,
A₃ représente un acide aminé choisi parmi la glycine, l'alanine, la valine, la leucine et l'isoleucine, de préférence la glycine,
A₄ représente un acide aminé choisi parmi l'arginine et l'histidine, de préférence l'arginine,
A₅ représente un acide aminé choisi parmi la glycine, l'alanine, la valine, la leucine et l'isoleucine, de préférence l'alanine,
A₆ représente un acide aminé choisi parmi la glycine, l'alanine, la valine, la leucine et l'isoleucine, de préférence la glycine.

Ainsi, des séquences peptidiques minimum particulièrement préférées sont les séquences:
KGPGRAKG (SEQ ID N°1)
KGFGRAKG (SEQ ID N°2)
KPGGRAKG (SEQ ID N°3)
   et
KFGGRAKG (SEQ ID N°4)

On préfère que la molécule porteuse comprenne au moins deux résidus choisis parmi les acides aminés à chaînes latérales basiques, notamment la lysine, les acides aminés à chaînes latérales chargés négativement, notamment l'acide glutamique ou l'acide aspartique, les acides aminés à chaînes latérales hydroxylés, notamment la sérine et la thréonine, et les acides aminés à chaînes latérales contenant du soufre, notamment la cystéine, la lysine étant D'une manière générale, la molécule porteuse selon l'invention a une masse moléculaire ne dépassant pas environ 10 kilodaltons, de préférence ne dépassant pas environ 8 kilodaltons. Les molécules porteuses préférées selon l'invention ont une masse moléculaire inférieure à environ 5 kilodaltons. La masse moléculaire minimum requise est généralement d'environ 1 plus ou moins 0,2 kilodalton.

Les épitopes peuvent consister en des épitopes quelconques de la molécule biologique à doser pour lesquels existent des anticorps, de préférence des anticorps spécifiques.

On peut citer pour la troponine l des anticorps décrits par Larue et al. *(Molec. Immunology, (1992), vol. 20 n° 29, pp. 271-278),* Bodor et al. *(Clin. Chem. (1992), vol 38 n° 11, pp. 2203-2214),* Granier et al *(Protein Science, (1997), vol. 6 suppl. 1, p. 61)* et dans la demande WO 94/15217. La majorité de ces anticorps sont disponibles dans le commerce (Hytest LTD-Turku, Finland). On préfère notamment les anticorps 11E12 et 8E1.

Les épitopes sont dénommés ci-après par E1 et E2, E1 étant différent de E2.

Les épitopes E1 et E2 peuvent être présents en un seul exemplaire chacun sur la molécule porteuse ou en plusieurs, notamment deux, trois ou quatre exemplaires.

Dans ce dernier cas, les deux exemplaires du même épitope peuvent être présents sur le même bras latéral ou sur deux bras latéraux différents.

De manière avantageuse, la synthèse des composés selon l'invention peut être contrôlée rigoureusement : pour s'assurer du nombre d'épitopes E1 ou E2 que l'on a accrochés sur la molécule porteuse, on pourra utiliser l'ouvrage suivant : Synthetic *Peptides A user's guide,* édité par Gregory A. Grant, (UWBC Biotechnological Resource Series, Richard R. Burgess series editor), 1992, au chapitre 4 : Evaluation of the finished product. Voir ci-après.

On préfère toutefois qu'un même bras latéral ne comporte pas plus de deux épitopes identiques ou différents.

Dans le cas où les épitopes sont présents à plus de deux exemplaires, on préfère que ceux-ci soient portés par autant de bras latéraux supplémentaires.

Lorsqu'ils sont présents en deux exemplaires sur les même bras latéraux, les épitopes E1 et E2 sont avantageusement séparés les un des autres par un groupe de liaison Z ("linker") qui peut être de nature peptidique ou non peptidique.

Aussi, le groupe de liaison Z peut représenter :
- une séquence peptidique de 1 à 40, de préférence 3 à 20 acides aminés, à condition toutefois que l'enchaînement formé par les épitopes et le groupe de liaison Z ne forment pas ensemble une partie de la séquence de la molécule biologique à doser, notamment la troponine I ;
- une chaîne aminoalkyl-(C₁-C₁₀) carbonyle linéaire ou ramifiée ;
- une construction mixte constituée d'au moins une séquence peptidique de 1 à 10 acides aminés et au moins une chaîne aminoalkyl(C₁-C₁₀)carbonyle linéaire ou ramifiée.

On considère que - E₁ - Z - E₂ - n'est pas une partie de la séquence de la molécule biologique à doser, notamment la troponine I, lorsque - E₁ - Z - E₂ - diffère d'un fragment donné de la séquence de la molécule à doser, par exemple la troponine I par substitution, délétion ou insertion non conservative d'au moins un acide aminé, de préférence de 2 acides aminés, plus particulièrement de 5 acides aminés.

Z peut notamment représenter
- une chaîne de formule :

   -NH-(CH₂)ₘ-CO-

   dans laquelle m représente un nombre entier de 1 à 10,
- ou une construction mixte de formules :

   -[pep₁-NH-(CH₂)ₘ-CO]ₚ-

   -[pep₁-NH-(CH₂)ₘ- CO-pep₂]ₚ-

   -[NH-(CH₂)ₘ-CO-pep₂]ₚ-

   dans lesquelles
   - m représente un nombre entier de 1 à 10,
   - p représente un nombre entier de 1 à 5, et
   - pep₁ et pep₂ identiques ou différents représentent une chaîne peptidique comportant de 2 à 10 acides aminés.

Les bras latéraux portant les épitopes comprennent de préférence aux extrémités N- et C-terminales des épitopes, ou de l'enchaînement formé par E₁-Z-E₂ des groupes supplémentaires respectivement Z₁ et Z₂.

Avantageusement, Z₁ représente :
- un atome d'hydrogène, un groupement acétyle, une séquence peptidique de 1 à 10 acides aminés, une séquence peptidique de 1 à 10 acides aminés N- terminale α acétylée, un groupement cystéinyle, biotinyle ou biocytinyle, une séquence peptidique de 1 à 10 acides aminés portant un reste cystéinyle, amino, hydroxyle, halogéno, carboxyle, biotinyle ou biocytinyle,
- une chaîne aminoalkyl(C₁-C₁₀)carbonyle linéaire ou ramifiée,
- une chaîne aminoalkyl(C₁-C₁₀)carbonyle linéaire ou ramifiée N-α-acétylée,
- une construction mixte constituée d'au moins une séquence peptidique de 1 à 10 acides aminés et au moins une chaîne aminoalkyl(C₁₋C₁₀)carbonyle linéaire ou ramifiée, ou
- une construction mixte constituée d'au moins une séquence peptidique de 1 à 10 acides aminés et au moins une chaîne aminoalkyl(C₁₋C₁₀)carbonyle linéaire ou ramifiée, portant un reste amino, halogéno, hydroxyle, carboxyle, biotinyle, biocytinyle ou cystéinyle.

Avantageusement Z₂ représente :
- un radical hydroxyle, un radical amino, une séquence peptidique de 1 à 10 acides aminés, une séquence peptidique de 1 à 10 acides aminés portant un groupement amino terminal, hydroxyle, carboxyle, halogéno, cystéinyle, biotinyle ou biocytinyle,
- une chaîne aminoalkyl(C₁-C₁₀)carbonyle linéaire ou ramifiée,
- une chaîne aminoalkyl(C₁-C₁₀)carbonyle linéaire ou ramifiée portant un radical hydroxyle ou un radical amino,
- une construction mixte constituée d'au moins une séquence peptidique de 1 à 10 acides aminés et au moins une chaîne aminoalkyl(C₁₋C₁₀)carbonyle linéaire ou ramifiée,
- une construction mixte constituée d'au moins une séquence peptidique de 1 à 10 acides aminés et au moins une chaîne aminoalkyl(C₁₋C₁₀)carbonyle linéaire ou ramifiée, portant un radical hydroxyle ou un radical amino.

Les bras latéraux sont liés à la molécule porteuse soit - directement, soit par l'intermédiaire d'un espaceur (« spacer ») qui peut être un groupement bifonctionnel, comprenant à ses extrémités deux fonctions réactives choisies pour réagir avec les groupements réactifs portés respectivement par la molécule porteuse et Z₁ ou Z₂.

On préfère que les bras latéraux comprennent des épitopes formés d'au plus 10, avantageusement d'au plus 6 acides aminés et que les groupes Z, et Z₁ et Z₂ soient formés d'acides aminés naturels et/ou non naturels.

Les composés synthétiques de l'invention peuvent être obtenus par un procédé comprenant les étapes suivantes :
- étape A : fourniture d'une molécule porteuse possédant une chaîne hydrocarbonée peptidique comprenant au moins deux groupes réactifs permettant le greffage de bras latéraux.
- étape B : éventuellement cyclisation de la molécule porteuse.
- étape C : fourniture de molécules formant les bras latéraux, comprenant au moins une fonction réactive avec le groupe réactif de la molécule porteuse.
- étape D : couplage des bras latéraux et de la molécule porteuse dans des conditions appropriées.

Lorsque les espaceurs (« spacers ») sont de nature polypeptidique, les étapes ci-dessus décrites peuvent avantageusement constituer en :
étape A : fourniture d'un oligopeptide ou polypeptide comprenant au moins deux résidus acides aminés comportant au moins deux groupes latéraux fonctionnels (par exemple : amino) identiques ou différents.
   Dans le cas d'un peptide cyclisé, les groupements fonctionnels sont choisis de façon à ce qu'il n'y ait pas d'interférence lors de la cyclisation, ou alors ceux-ci sont protégés de façon temporaire à l'aide d'un groupe protecteur usuel.
étape B : cyclisation éventuelle du peptide soit directement, soit par l'intermédiaire d'un agent de couplage.
   Par exemple, dans le cas de la formation d'une liaison amide, le couplage peut être réalisé par l'intermédiaire du couple hexafluorophosphate de benzotriazolyl-oxy-tris -(diméthylamine)phosphonium/1-hydroxybenzotriazole (BOP/HOBt) en présence d'une base.
étape C : fourniture des peptides des bras latéraux non protégés, chacun portant un groupement fonctionnel susceptible de réagir directement ou par l'intermédiaire d'un agent de couplage ; puis
étape D : couplage dudit oligopeptide ou polypeptide avec les peptides formant les bras latéraux de manière à obtenir le composé synthétique selon l'invention.

Dans une forme de réalisation préférée, les fonctions réactives de la molécule porteuse sont des fonctions amino, activées par l'intermédiaire d'un groupement bifonctionnel ₛSMCC.

Dans l'étape D, la liaison entre un bras latéral et la molécule porteuse est réalisée par une réaction de substitution nucléophile entre un résidu -SH du peptide dudit bras latéral sur un groupement maléimide de la molécule porteuse.

La molécule porteuse et les bras latéraux sont obtenus par des procédés classiques de la synthèse organique et/ou de la chimie des peptides, le cas échéant.

Les peptides de la molécule porteuse et des bras latéraux portant les épitopes peuvent être obtenus par synthèse en phase solide selon des méthodes classiques décrits par R.B. Merrifield, *J. Amer. Chem. Soc. (1963), 85, pp. 2149-2154 ;* R.C. Sheppard, dans « *Peptides 1971 », Nesvadba H (ed.) North Holland, Amsterdam, pp. 111 ;* E. Atherton et R.L. Sheppard, dans *« Solid phase peptide synthesis, a practical approach », IRL PRESS, (1989), Oxford University Press, pp. 25-34.*

Comme synthétiseur automatique, on peut utiliser le synthétiseur « 9050 Plus Pep Synthesizer » de Millipore, "Pioneer" de Perspective ou le synthétiseur "433A" de ABI.

Les peptides peuvent également être obtenus par synthèse en phase homogène.

Le support solide utilisé pour les synthèses doit être compatible avec la technique et la chimie utilisées. Par exemple, pour une synthèse sur le synthétiseur « 9050 Plus pep. Synthesizer » il est recommandé d'utiliser une résine adaptée à la technique dite « en flux continu » ; les résines PEG PS répondent à ces critères. Ces supports sont constitués d'un bras espaceur (« spacer ») à base de polyéthylène glycol (PEG) situé entre le groupement fonctionnel du polystyrène des billes et le point d'accrochage du premier acide aminé. La nature de ce point d'ancrage peut varier selon la fonction C-terminale choisie. Par exemple, pour un peptide sous forme d'amide, on pourra prendre une résine de type PAL PEG PS.

Les résines de Novabiochem répondent aussi aux critères nécessaires à la synthèse en phase solide en flux continu. Elles présentent en outre l'avantage de pouvoir libérer le peptide après synthèse dans des conditions acides dites douces, ce qui permet d'obtenir des fragments peptidiques dont les chaînes latérales des acides aminés sont encore protégées par des groupements chimiques.

La résine de départ et les acides aminés utilisés comme matière première sont des produits disponibles dans le commerce (PerSeptive-Biosystem, Perkin-Elmer, Novabiochem).

Les groupements protecteurs de chaînes latérales qui peuvent être utilisés sont représentés au tableau I :

**Tableau I : groupements protecteurs.**

| Acides aminés | Groupements protecteurs |
|---|---|
| Arginine | Pentaméthyl-2,2,4,6,7-dihydrobenzofuran-5-sulfonyle (Pbf) |
| Asparagine, Glutamine | Trityle (Trt) |
| Acide glutamique | Ester tert-butyle (otBu) |
| Thréonine, Tyrosine, Sérine | Ether tert-butyle (tBu) |
| Lysine | Tert-butyloxycarbonyle (Boc) |

La protection temporaire de la fonction amine primaire en α des acides aminés peut être effectuée à l'aide du groupement 9-fluorénylméthyloxylcarbonyle (Fmoc). La déprotection peut être effectuée par une solution de pipéridine à 20% dans la diméthylformamide.

Pour le couplage, on utilise de préférence un excès de diisopropylcarbodiimide (DIPCDI) et de 1-hydroxy-benzotriazole (HOBt).

Pour les peptides cycliques constituant la molécule porteuse, on utilise de préférence la résine Fmoc Ala Nova Syn

Après synthèse, la résine est lavée avec des solvants organiques, (diméthylformamide, puis dichlorométhane) séchée sous vide puis traitée par une solution à base d'acide.

Les peptides ainsi isolés sont ensuite précipités et rincés à l'éther.

Pour des molécules porteuses formées de peptides cycliques, on peut, par exemple, réaliser une cyclisation dite "tête queue". Pour cette étape, le peptide est dissous dans du DMF, l'agent de couplage étant le couple BOP/HOBt en présence d'une base comme la diisopropylènamine (DIEA), ou la N-méthylmorpholine (NMM). Après extraction, le peptide cyclisé est précipité puis lavé par une solution à base d'éther.

Le peptide ainsi obtenu est ensuite traité par une solution d'acide trifluoroacétique, puis de nouveau précipité et rincé à l'éther.

Pour des molécules porteuses de nature peptidique non cycliques, on utilise une résine PALPEG PS. Après synthèse, la résine est lavée sur des solvants organiques, (diméthylformamide, dichlorométhane), séchée sous vide, puis traitée par une solution à base d'acide trifluoroacétique refroidie à 0°C et contenant ses "scavengers" appropriés. On peut utiliser par exemple le réactif K, contenant 82 % d'acide trifluoroacétique, 5 % de phénol, 5 % d'eau, 5 % de thioanisole et 3 % d'éthane dithiol.

Les peptides formant les bras latéraux peuvent être obtenus en particulier comme cela est décrit dans la demande WO 98/24 816.

Les peptides synthétiques ainsi isolés sont ensuite précipités à l'éther.

Les composés synthétiques cycliques ou linéaires sont ensuite purifiés par chromatographie liquide en phase inverse et leur pureté est déterminée par spectrométrie de masse. Comme phase, on peut utiliser, par exemple, la phase Bondapak C-18. Les peptides sont élués au moyen d'un gradient linéaire entre deux solutions tampon, la première essentiellement aqueuse (par exemple eau-TFA 0,1 %) et la seconde plutôt organique (par exemple un mélange contenant de l'acétonitrile 60 %, eau 40 % et TFA 0,08 %). Les fractions pures collectées sont rassemblées, concentrées sous vide et lyophylisées et leur pureté est contrôlée par spectrométrie de masse, comme cela est indiqué dans l'ouvrage suivant : Synthetic Peptides *A user's guide,* édité par Gregory A. Grant, (UWBC Biotechnological Resource Series, Richard R. Burgess series editor), 1992, au chapitre 4: Evaluation of the finished product.

Les fonctions des chaînes latérales de certains acides aminés (par exemple, de la lysine, de l'arginine, de la cystéine, etc..) constitutifs de l'oligopeptide ou du polypeptide porteur sont ensuite activées par un groupement bifonctionnel permettant la liaison de façon spécifique, ou non, d'un ou plusieurs peptides (formant les bras latéraux). Comme exemple de chaîne latérale susvisée pouvant être utilisée dans ce type de liaison, on peut citer les chaînes portant des groupes amino (primaire/secondaire), carboxyle, thiol, hydroxyde, aldéhyde, etc ...

Comme exemple de composés comprenant des groupements bifonctionnels, on peut utiliser les composés suivants :
- BS₃ : bis(sulfosuccinimidyl)subérate ;
- ₛSMCC : (sulfosuccinimidyl 4-N-maléimidométhyl) cyclo-hexane 1-carboxylate.
- BMH : bis-maléido hexane
- DSG : Disuccinimidyl glutarate
- 5MBS : m-maleimidobenzoyl-N-hydroxy sulfo succimide ester
- MPBH : 4-(4-N-maleimidophényl)butyric acide hydroxide
- EDC : 1-ethyl-3-(3-diméthylaminopropylate) carbodiimide

En utilisant la technique de spectrométrie de masse suscitée aux composés synthétiques finals selon l'invention, l'homme du métier peut vérifier le degré de pureté d'un tel composé. En outre, il peut contrôler de façon claire et non ambiguë que la masse moléculaire dudit composé effectivement mesurée par spectrométrie correspond bien à la masse moléculaire attendue au vu des masses moléculaires des molécules de départ (molécule porteuse, épitopes, ...). La présente invention permet donc à l'homme du métier de contrôler précisément les synthèses qu'il réalise et de savoir exactement ce qu'il fait.

L'invention a également pour objet des composés comprenant deux épitopes différents de la troponine I tels qu'ils ont été définis précédemment.

Comme épitopes de la troponine I, on peut citer notamment les séquences peptidiques TEPH (SEQ ID N°5), ALLGAR (SEQ ID N°6) et FAEL (SEQ ID N°7) ou les séquences comprenant ces dernières.

L'invention a également pour objet des compositions contenant les composés de l'invention comprenant au moins deux épitopes de la troponine I.

Il s'agit, de préférence, de solutions aqueuses ou de compositions constituées de composés synthétiques biépitopiques, tels qu'ils ont été décrits précédemment, dans une solution tampon. Comme solution tampon, on peut utiliser, par exemple, une solution tampon phosphate (KH₂PO₄/K₂HPO₄ pH = 6,5 - 7,5) contenant du ®Kathon et de l'albumine sérique bovine (BSA), ou du - ®Kathon, du ®Régilait et de l'EDTA, ou du ®Kathon, du ®Plasmion et éventuellement de l'EDTA, ou du ®Kathon, de la caséine et de l'EDTA.

On peut utiliser également une solution tampon succinate (pH = 5 - 6) ou Tris HCl (pH = 7,5 - 8,5) contenant du ®Kathon et de l'albumine sérique bovine, ou du ®Kathon, du ®Régilait et de l'EDTA ou du ®Kathon, du ®Plasmion et éventuellement de l'EDTA, ou du ®Kathon, de la caséine et de l'EDTA.

Des solutions tampon contenant de la glycine, du ®Kathon, du ®Régilait et de l'EDTA peuvent être également utilisées.

On préfère utiliser une solution tampon succinate ou phosphate, contenant du ®Kathon, du ®Régilait et de l'EDTA.

Le ®Kathon, agent anti-bactérien commercialisé par la Société Rohm et Haas, est constitué de 5-chloro 2-méthyl 4-isothiazolin-3-one et de 2-méthyl 4-isothiazolin-3-one (1,5%).

Le ®Régilait est un lait en poudre écrémé commercialisé par la société Régilait (France).

Le ®Plasmion est commercialisé par les Laboratoires Bellon (France) et est constitué de gélatine fluide modifiée (30 g/l), NaCl (5,382 g/l), MgCl (143 mg/l), KCI (373 mg/l), lactate de sodium (3,360 g/l), dans l'eau.

Les solutions tampons suivantes sont particulièrement préférées :
- solution tampon succinate 0,1 M (pH = 6) contenant du ®Kathon (à 0,2%), du ®Régilait (0,05 - 2%) et de l'EDTA 2mM,
- solution tampon succinate 0,1 M (pH = 6) contenant du ®Kathon (à 0,2%) de la caséine (0,01 - 0,5%) et de l'EDTA 2mM,
- solution tampon succinate 0,1 M (pH = 6) contenant du ®Kathon (à 0,2%) et de la BSA à 1%,
- solution tampon phosphate KH₂PO₄/K₂HPO₄ 0,1 M (pH = 7,5) contenant du ®Kathon (à 0,2%) du ®Régilait (0,05 - 0,5%) et de l'EDTA 2mM,
- solution tampon phosphate KH₂PO₄/K₂PO₄ 0,1 M (pH = 7,5) contenant du ®Kathon (à 0,2%), de la caséine (0,01 - 0,1%) et de l'EDTA 2mM,
- solution tampon phosphate KH₂PO₄/K₂PO₄ 0,1 M (pH = 7,5) contenant du ®Kathon (à 0,2%), et de la BSA à 1%.

Des compositions, contenant du plasma, ou du sérum, et un composé de l'invention défini ci-dessus, font aussi partie de la présente invention.

Des procédés d'immunodosages utilisant comme étalons ou témoins les composés tels qu'ils ont été définis ci-dessus font également partie de l'invention.

L'invention vise aussi des trousses de mise en oeuvre d'immunodosages qui incluent au moins un composé biépitopique défini ci-dessus ou au moins une composition qui contient un peptide biépitopique défini ci-dessus.

Les exemples suivants illustrent l'invention et sont donnés à titre non limitatif.

### EXEMPLE 1 :

Préparation de composés biépitopiques synthétiques selon l'invention.

### A. Préparation de la molécule porteuse.

On prépare un peptide cyclique de formule : le code étant le code à une lettre.

Ce peptide est synthétisé en phase solide par la technique mise au point en 1963 par Merrifield (J. Am. Chem. Soc. 1963, 85, pp 2149-2154) comme cela a été décrit précédemment.

Pour la synthèse du composé ci-dessus, ont été utilisés, comme synthétiseur, le synthétiseur 9050 Plus Synthesizer et, comme résine, la résine Fmoc Ala Nova Syn. Les différentes étapes de la synthèse sont résumées dans le tableau Il suivant :

**Tableau II : Etapes de la synthèse.**

| Résidu d'acide aminé | Protection NH₂ | Protection latérale | Méthode de couplage | Nombre d'équivalents/ Durée/ Nombre de couplages |
|---|---|---|---|---|
| Ser | Fmoc | tBu | DIPCDI/HOBt | 5 eq/30'/Dc* |
| Gly | " | - | " | " |
| Pro | " | - | " | " |
| Gly | " | - | " | " |
| Lys | " | Boc | " | " |
| Ala | " | - | " | " |
| Arg | " | Pbf | " | " |
| Gly | " | - | " | " |
| Pro | " | - | " | " |
| Gly | " | - | " | " |
| Lys | " | Boc | " | " |

| | | | | |
|---|---|---|---|---|
| Dc* signifie double couplage. Un double couplage peut être effectué pour augmenter la pureté du peptide. | | | | |

A la fin de la synthèse, la résine est lavée avec du diméthylformamide, puis du dichlorométhane et séchée sous vide.

Ensuite, la résine est traitée avec une solution contenant de l'acide acétique, du méthanol et du dichlorométhane (5/1/4 V/V/V).

La résine est isolée du peptide en solution par filtrage. Le filtrat est concentré, et le peptide, isolé par précipitation à l'éther refroidi. On obtient ainsi 0,145 g du composé.

Le peptide est ensuite cyclisé de la manière suivante :
100 mg du composé sont dissous dans 18 ml de diméthylformamide auxquels sont ajoutés successivement 2 équivalents de BOP, 2 équivalents d'HOBt, et 5 équivalents de DIEA.

Après deux heures de réaction, le peptide, cyclisé, est extrait en ampoule à décanter.

La phase organique est ensuite séchée sur Na₂SO₄ puis concentrée au ®Rotavapor, ou à l'aide de tout autre évaporateur rotatif approprié. Le peptide cyclisé est ensuite précipité dans un mélange d'éther, d'acétate d'éthyle et d'hexane (6/3/1) V/V/V), pendant 15 heures à 0°C. On obtient 70 mg du peptide cyclisé, c'est à dire 70 mg de molécule porteuse.

### B. Préparation de composés synthétiques biépitopiques selon l'invention.

8 mg de molécule porteuse (i.e. du peptide cyclisé ci-dessus) dissous dans un mélange DMF/tampon (1/3 / 2/3) sont activés par 6 équivalents de ₛSMCC ajoutés goutte à goutte. La réaction est maintenue sous agitation pendant environ 45 minutes à 18 - 25 °C. La molécule porteuse activée est séparée de l'excès de ₛSMCC par HPLC. Les fractions pures sont rassemblées et lyophilisées. On obtient 4,5 mg de molécule porteuse activée (rendement ≈ 45 %)
2 mg de molécule porteuse activée sont dissous dans un tampon PBS pH 6,5. A cette solution sont ajoutés 2 équivalents (soit 8 mg) du peptide TRP 1116 Ac suivant:
   Ac-GSSNYRAYA―(TEPH)―AKK-Hx-PLELAGLG-(FAEL)-QDLCRQ-NH₂ (SEQ ID N°9)

Les séquences peptidiques entre parenthèses représentent des épitopes de la troponine I.

La réaction est maintenue sous agitation pendant quatre heures à 18 - 25 °C.

Le composé selon l'invention ainsi obtenu est ensuite dessalé (HPLC/dialyse).

Sa masse moléculaire est évaluée par spectrométrie de masse (selon le protocole indiqué dans l'ouvrage Synthetic Peptides *A user's guide,* édité par Gregory A. Grant, (UWBC Biotechnological Resource Series, Richard R. Burgess series editor), 1992, au chapitre 4 : Evaluation of the finished product.

De la même manière, on a préparé les composés selon l'invention PEP 5, PEP 9 et PEP 10 de formules suivantes :
- composé PEP 5 :
- composé PEP 9 :
- composé PEP 10 :
en deux lots PEP 10 P1 et PEP 10 P2.

Les résultats de stabilité et de linéarité de dilution des composés selon l'invention sont donnés ci-après :

### EXEMPLE 2 :

Stabilité des composés selon l'invention.

La stabilité des composés PEP5, PEP9 et PEP10 a été évaluée en essai ELISA, à lecture en chimioluminescence (signal exprimé en Unités Relatives de Luminescence ou URL), à l'aide de l'automate Access®, de la société Beckman, distribué par Bio-Rad (Marnes la Coquette, France), puis comparée à celle d'un composé biépitopique de référence, non cyclisé, de l'art antérieur (TRP 1116 Ac) analysé de la même manière.

Les résultats sont présentés dans le tableau III ci-après.

**Tableau III**

| COMPOSE | DILUABILITE* (TAMPON ***) | STABILITE** LIQUIDE A +4°C EN TAMPON EXPRIMEE PAR LE RAPPORT « signal à JO + X /signal à JO » | | | | | |
|---|---|---|---|---|---|---|---|
| | | JO + 24H/JO | JO + 1 mois/JO | JO + 3 mois/JO | JO + 6 mois/JO | JO + 9 mois/JO | JO + 12 mois/JO |
| TRP 1116 Ac | NON | 0,77 | - | - | - | - | - |
| PEP 5 | OUI | | 0,94 | 0,89 | 1,06 | 1,04 | 1,03 |
| PEP 9 | OUI | | 1,01 | 0,94 | 0,95 | ND | ND |
| PEP 10 P1 | OUI | | 1,12 | 1,07 | ND | ND | ND |
| P2 | OUI | | 1,13 | 1,13 | ND | ND | ND |

| | | | | | | | |
|---|---|---|---|---|---|---|---|
| * Norme d'acceptation des dilutions : le Rapport signal expérimental /signal théorique à une dilution donnée doit être égal à 1.00 ± 0.2, pour que le composé soit diluable. Les dilutions donnant des signaux inférieurs à 100 000 unités URL ne doivent pas être prises en compte. ** Norme d'acceptation des stabilités : pour que la stabilité à un jour X après JO soit acceptable, il faut obtenir un rapport signal à JO + X / signal JO qui soit égal à 1.00 ± 0.2. *** Tampon succinate 0.1 M, pH 6.0, contenant du Kathon à 0.2%, du Régilait à 0.2% et de l' EDTA 2mM (ou bien tampon succinate 0.1 M, pH 6.0, contenant du Kathon à 0.2% et de la BSA à 1 %). ND = non déterminé. | | | | | | | |

Le composé de l'art antérieur TRP1116 Ac n'est pas stable à JO + 24 heures et au-delà, à + 4°C.

Les composés selon l'invention PEP5, PEP9 et PEP10 sont tous stables à + 4°C après 3 mois, voire 6 mois pour PEP 9, et 12 mois pour PEP 5.

### EXEMPLE 3 :

### Résultats des linéarités des courbes de dilution.

Les résultats sont représentés dans le tableau 5 ci-après.
Norme d'acceptation des dilutions : le Rapport signal expérimental / signal théorique à une dilution donnée doit être égal à 1.00 ± 0.2, pour que le composé soit diluable. Les dilutions donnant des signaux inférieurs à 100 000 unités URL ne doivent pas être prises en compte.

Résultats exprimés en unités de signal (URL)

**Tableau 5**

| | Dilution | Signal expérimental (URL) | Signal théorique (URL) | Signal expérimental /signal théorique |
|---|---|---|---|---|
| Composé | 1/10 000 | 11 039 697 | | |
| TRP 1116 Ac | 1/20 000 | 3 779 387 | 5 519 849 | 0,68 |
| | 1/40 000 | 1 573 457 | 2 759 924 | 057 |
| | 1/50 000 | 1 125 417 | 2 207 939 | 0,51 |
| | 1/100 000 | 471 714 | 1 103 970 | 0,43 |
| | 1/300 000 | 132 054 | 367 990 | 0,36 |
| Composé | 1/50 000 | 6 175 137 | | |
| PEP 5 | 1/100 000 | 3 175 837 | 3 087 S69 | 1,03 |
| | 1/200000 | 1 513 547 | 1 543 784 | 0,98 |
| | 1/500 000 | 547 444 | 617 514 | 0,89 |
| | 1/1000000 | 251 103 | 308 757 | 0,81 |
| | | | | |

| | Dilution | Signal expérimental (URL) | Signal théorique (URL) | Signal expérimental /signal théorique |
|---|---|---|---|---|
| Composé | 1/50000 | 6 416 384 | | |
| PEP 9 | 1/100 000 | 3411124 | 3208192 | 1,06 |
| | 1/200000 | 1 685 334 | 1 604 096 | 1,05 |
| | 1/500 000 | 655 134 | 641 638 | 1,02 |
| | 1/1000000 | 321 101 | 320819 | 1,00 |
| | | | | |

| | Dilution | Signal expérimental (URL) | Signal théorique (URL) | Signal expérimental /signal théorique |
|---|---|---|---|---|
| Composé | 1/50 000 | 4 786 295 | | |
| PEP 10 P1 | 1/100000 | 2 433 915 | 2 393 148 | 1,02 |
| | 1/200000 | 1 230 715 | 1 196574 | 1,03 |
| | 1/500 000 | 455 405 | 478 630 | 0,95 |
| | 1/1000000 | 215 853 | 239315 | 0,90 |
| Composé | 1/50 000 | 4 569 989 | | |
| PEP 10 P2 | 1/100000 | 2 287 659 | 2 284 995 | 1,00 |
| | 1/200 000 | 1 208 499 | 1 142 497 | 1,06 |
| | 1/500 000 | 407 015 | 456 999 | 0,89 |
| | 1/1000 000 | 192 408 | 228 499 | 0,84 |

Les courbes de dilution des composés selon l'invention PEP 5, PEP 9 et PEP 10 présentent encore une bonne linéarité, même aux fortes dilutions, contrairement au peptide TRP 1116 Ac.

En conséquence, des résultats fiables peuvent être obtenus avec les composés selon l'invention : non seulement ceux-ci s'avèrent être d'une plus grande stabilité que des composés de l'art antérieur, mais encore ils permettent d'obtenir une courbe de dilution linéaire. En d'autres termes, ils résolvent le problème posé.

## Revendications

1. Composé synthétique comprenant :
- une molécule porteuse possédant une chaîne hydrocarbonée peptidique formée
a) par des enchaînements de 7 à 50 acides aminés formant une chaîne hydrocarbonée ouverte comprenant au moins un enchaînement introduisant une rigidité dans la molécule de sorte que les enchaînements formés par les atomes de la chaîne hydrocarbonée ne soient plus tous en libre rotation ; ou
b) par des enchaînements de 8 à 50 acides aminés formant un cycle ; ladite chaîne hydrocarbonée comprenant au moins deux résidus sélectionnés dans le groupe constitué des acides aminés à chaînes latérales basiques, des acides aminés à chaînes latérales chargées négativement, des acides aminés à chaînes latérales hydroxylées et des acides aminés à chaînes latérales contenant du soufre, permettant le greffage de bras latéraux sur ladite chaîne hydrocarbonée; et
- au moins deux épitopes de troponine I différents portés par lesdits bras latéraux greffés sur ladite molécule porteuse.

2. Composé synthétique selon la revendication 1, dans lequel lesdits deux résidus permettant le greffage de bras latéraux sur ladite chaîne hydrocarbonée sont des lysines.

3. Composé synthétique selon la revendication 1 ou 2, dans lequel ladite chaîne hydrocarbonée comprend un résidu proline.

4. Composé synthétique selon l'une quelconque des revendications précédentes, dans lequel chacun desdits bras latéraux porte au moins deux épitopes de troponine I différents.

5. Composé synthétique selon la revendication 4, dans lequel chacun desdits bras latéraux porte deux épitopes de troponine I différents.

6. Composé synthétique selon l'une quelconque des revendications précédentes, dans lequel deux bras latéraux consécutifs portant les épitopes sont portés par des acides aminés espacés l'un de l'autre par un nombre 2n + 1 d'acides aminés, où n représente un nombre entier et est compris entre 1 et 6.

7. Composé synthétique selon l'une quelconque des revendications précédentes, dans lequel la chaîne hydrocarbonée de nature peptidique comprend la séquence :
X₁-A₁-A₂-A₃-A₄-A₅-X₂-A₆ dans laquelle
- X₁ et X₂, identiques ou différents, portent les bras latéraux et sont choisis parmi un acide aminé à chaîne latérale basique, un acide aminé à chaîne latérale chargée négativement, un acide aminé à chaîne latérale hydroxylée, et un acide aminé à chaîne latérale soufrée, X₁ et X₂ représentant de préférence la lysine,
- A₁ et A₂ représentent un acide aminé choisi parmi la proline, la phénylalanine, la glycine, l'alanine, la valine, la leucine et l'isoleucine, sous réserve que l'un de A₁ et A₂ soit choisi parmi la proline et la phénylalanine,
- A₃ représente un acide aminé choisi parmi la glycine, l'alanine, la valine, la leucine et l'isoleucine, de préférence la glycine,
- A₄ représente un acide aminé choisi parmi l'arginine et l'histidine, de préférence l'arginine,
- A₅ représente un acide aminé choisi parmi la glycine, l'alanine, la valine, la leucine et l'isoleucine, de préférence l'alanine, et
- A₆ représente un acide aminé choisi parmi la glycine, l'alanine, la valine, la leucine et l'isoleucine, de préférence la glycine.

8. Composé synthétique selon la revendication 7, dans lequel la chaîne hydrocarbonée de nature peptidique comprend une séquence sélectionnée dans le groupe constitué des séquences :
-KGPGRAKG-
-KGFGRAKG-
-KPGGRAKG- et
-KFGGRAKG-.

9. Composé synthétique selon l'une quelconque des revendications précédentes, dans lequel chaque épitope comprend au moins une séquence sélectionnée dans le groupe constitué des séquences :
-TEPH ;
-ALLGAR ; et
-FAEL.

10. Composé synthétique selon l'une quelconque des revendications précédentes, dans lequel la molécule porteuse est sélectionnée dans le groupe constitué de : et Ac-KGPGRAKGPGSA-NH₂.

11. Utilisation d'un composé synthétique selon l'une des revendications 1 à 10, comme étalon ou témoin pour des immunodosages de troponine I.

12. Composition contenant au moins un composé synthétique selon l'une quelconque des revendications 1 à 10, en solution dans l'eau, dans le plasma ou dans une solution tampon.

13. Composition selon la revendication 12, **caractérisée en ce que** la solution tampon est choisie parmi les solutions tampon phosphate, succinate et Tris HCl.

14. Composition selon la revendication 13, dans laquelle la solution tampon est sélectionnée dans le groupe constitué de solutions suivantes :
- solution tampon succinate (pH = 5-6) contenant du ®Kathon, du Régilait et de l'EDTA,
- solution tampon succinate (pH = 5-6) contenant du ®Kathon, de la caséine et de l'EDTA,
- solution tampon succinate (pH = 5-6) contenant du ®Kathon et de la BSA,
- solution tampon phosphate (KH₂PO₄/K₂HPO₄ pH = 6,5-7,5) contenant du ®Kathon, du Régilait et de l'EDTA,
- solution tampon phosphate (KH₂PO₄/K₂HPO₄ 0,1 M, pH = 6,5-7,5) contenant du ®Kathon, de la caséine et de l'EDTA.
- solution tampon phosphate KH₂PO₄/K₂PO₄ 0,1 M (pH = 6,5-7,5) contenant du ®Kathon et de la BSA.

15. Procédé d'immunodosage utilisant comme étalon ou témoin au moins un composé synthétique selon l'une quelconque des revendications 1 à 10.

16. Procédé selon la revendication 15, dans lequel l'immunodosage est de type sandwich.

17. Trousse d'immunodosage comprenant :
- au moins un composé synthétique selon l'une quelconque des revendications 1 à 10 ou une composition selon l'une quelconque des revendications 12 à 14;
- au moins un réactif usuel de mise en oeuvre d'un immunodosage.

## Claims

1. A synthetic compound comprising:
- a carrier molecule having a peptide hydrocarbon chain formed
a) by linkages of 7 to 50 amino acids forming an open hydrocarbon chain comprising at least one linkage introducing rigidity into the molecule such that the linkages formed by the atoms of the hydrocarbon chain are no longer all able to rotate freely; or
b) by linkages of 8 to 50 amino acids forming a cycle; said hydrocarbon chain comprising at least two residues selected from the group made up of amino acids with basic side chains, of amino acids with negatively charged side chains, of amino acids with hydroxylated side chains and of amino acids with side chains containing sulfur, allowing the grafting of side arms onto said hydrocarbon chain; and
- at least two different troponin I epitopes carried by said side arms grafted onto said carrier molecule.

2. A synthetic compound according to claim 1, in which said two residues allowing the grafting of side arms onto said hydrocarbon chain are lysines.

3. A synthetic compound according to claim 1 or claim 2, in which said hydrocarbon chain comprises a proline residue.

4. A synthetic compound according to any one of the preceding claims, in which each of said side arms carries at least two different troponin I epitopes.

5. A synthetic compound according to claim 4, in which each of said side arms carries two different troponin I epitopes.

6. A synthetic compound according to any one of the preceding claims, in which two consecutive side arms carrying the epitopes are carried by amino acids spaced from one another by a number 2n + 1 of amino acids, where n represents an integer and is between 1 and 6.

7. A synthetic compound according to any one of the preceding claims, in which the peptide-type hydrocarbon chain comprises the sequence:
X₁-A₁-A₂-A₃-A₄-A₅-X₂-A₆ in which
- X₁ and X₂, identical or different, carry the side arms and are selected from among an amino acid with a basic side chain, an amino acid with a negatively charged side chain, an amino acid with a hydroxylated side chain, and an amino acid with a sulfur-containing side chain, X₁ and X₂ preferably representing lysine,
- A₁ and A₂ represent an amino acid selected from among proline, phenylalanine, glycine, alanine, valine, leucine and isoleucine, on condition that one of A₁ and A₂ is selected from among proline and phenylalanine,
- A₃ represents an amino acid selected from among glycine, alanine, valine, leucine and isoleucine, preferably glycine,
- A₄ represents an amino acid selected from among arginine and histidine, preferably arginine,
- A₅ represents an amino acid selected from among glycine, alanine, valine, leucine and isoleucine, preferably alanine, and
- A₆ represents an amino acid selected from among glycine, alanine, valine, leucine and isoleucine, preferably glycine.

8. A synthetic compound according to claim 7, in which the peptide-type hydrocarbon chain comprises a sequence selected from the group made up of the sequences:
-KGPGRAKG-
-KGFGRAKG-
-KPGGRAKG- and
-KFGGRAKG-.

9. A synthetic compound according to any one of the preceding claims, in which each epitope comprises at least one sequence selected from the group made up of the sequences:
- TEPH;
- ALLGAR; and
- FAEL.

10. A synthetic compound according to any one of the preceding claims, in which the carrier molecule is selected from the group made up of: and **Ac-KGPGRAKGPGSA-NH**_{**2**}**.**

11. Use of a synthetic compound according to any one of claims 1 to 10, as a standard or control for troponin I immunoassays.

12. A composition containing at least one synthetic compound according to any one of claims 1 to 10, in solution in water, in plasma or in a buffer solution.

13. A composition according to claim 12, **characterised in that** the buffer solution is selected from among phosphate, succinate and tris-HCl buffer solutions.

14. A composition according to claim 13, in which the buffer solution is selected from the group made up of the following solutions:
- succinate buffer solution (pH = 5-6) containing ^{®}Kathon, Régilait powdered milk and EDTA,
- succinate buffer solution (pH = 5-6) containing ^{®}Kathon, casein and EDTA,
- succinate buffer solution (pH = 5-6) containing ^{®}Kathon and BSA,
- phosphate buffer solution (KH₂PO₄/K₂HPO₄ pH = 6.5-7.5) containing ^{®}Kathon, Régilait powdered milk and EDTA,
- phosphate buffer solution (KH₂PO₄/K₂HPO₄ 0.1 M, pH = 6.5-7.5) containing ^{®}Kathon, casein and EDTA,
- phosphate buffer solution KH₂PO₄/K₂PO₄ 0.1 M (pH = 6.5-7.5) containing ^{®}Kathon and BSA.

15. An immunoassay method using as standard or control at least one synthetic compound according to any one of claims 1 to 10.

16. A process according to claim 15, in which the immunoassay is of the sandwich type.

17. An immunoassay kit comprising:
- at least one synthetic compound according to any one of claims 1 to 10 or a composition according to any one of claims 12 to 14;
- at least one conventional reagent for implementation of an immunoassay.

## Patentansprüche

1. Synthetische Verbindung, umfassend:
- ein Trägermolekül, das eine peptidische Kohlenwasserstoffkette besitzt, gebildet
a) durch Verknüpfungen von 7 bis 50 Aminosäuren, die eine offene Kohlenwasserstoffkette bilden, welche wenigstens eine Verknü-pfung umfasst, die Steifigkeit in das Molekül derart einführt, dass die durch die Atome der Kohlenwasserstoffkette gebildeten Verknüpfungen nicht mehr alle in freier Rotation sind; oder
b) durch Verknüpfungen von 8 bis 50 Aminosäuren, die einen Ring bilden, wobei diese Kohlenwasserstoffkette wenigstens zwei Reste umfasst, ausgewählt in der Gruppe, welche durch Aminosäuren mit basischen Seitenketten, Aminosäuren mit negativ geladenen Seitenketten, Aminosäuren mit hydroxylierten Seitenketten und Aminosäuren mit Schwefel enthaltenden Seitenketten gebildet wird, welche die Pfropfung der Seitenarme auf dieser Kohlenwasserstoffkette erlauben; und
- wenigstens zwei verschiedene Epitope für Troponin I, die auf diesen gepfropften Seitenarmen auf diesem Trägermolekül getragen sind.

2. Synthetische Verbindung gemäß Anspruch 1, in welcher diese zwei Reste, die das Pfropfen der Seitenarme auf dieser Kohlenwasserstoffkette erlauben, Lysine sind.

3. Synthetische Verbindung gemäß Anspruch 1 oder 2, in welcher diese Kohlenwasserstoffkette einen Prolinrest umfasst.

4. Synthetische Verbindung gemäß irgendeinem der vorhergehenden Ansprüche, in welcher jeder dieser Seitenarme wenigstens zwei verschiedene Epitope für Troponin I trägt.

5. Synthetische Verbindung gemäß Anspruch 4, in welcher jeder dieser Seitenarme zwei verschiedene Epitope von Troponin I trägt.

6. Synthetische Verbindung gemäß irgendeinem der vorhergehenden Ansprüche, in welcher zwei aufeinanderfolgende Seitenarme, welche die Epitope tragen, durch Aminosäuren getragen werden, die voneinander durch eine Zahl von 2n + 1 Aminosäuren getrennt sind, worin n eine ganze Zahl darstellt und zwischen 1 und 6 liegt.

7. Synthetische Verbindung gemäß irgendeinem der vorhergehenden Ansprüche, in welcher die Kohlenwasserstoffkette von peptidischer Art die Folge umfasst:
X₁-A₁-A₂-A₃-A₄-A₅-X₂-A₆,
in welcher -X₁ und -X₂, gleich oder verschieden, die Seitenarme tragen und ausgewählt sind unter einer Aminosäure mit basischer Seitenkette, einer Aminosäure mit negativ geladener Seitenkette, einer Aminosäure mit hydroxylierter Seitenkette und einer Aminosäure mit Schwefel enthaltender Seitenkette, wobei X₁ und X₂ bevorzugt Lysin darstellen,
- A₁ und -A₂ eine Aminosäure darstellen, ausgewählt unter Prolin, Phenylalanin, Glycin, Alanin, Valin, Leucin und Isoleucin, mit der Maßgabe, dass eines von A₁ und A₂ unter Prolin und Phenylalanin ausgewählt ist,
- A₃ eine Aminosäure darstellt, ausgewählt unter Glycin, Alanin, Valin, Leucin und Isoleucin, bevorzugt Glycin,
- A₄ eine Aminosäure darstellt, ausgewählt unter Arginin und Histidin, bevorzugt Arginin,
- A₅ eine Aminosäure darstellt, ausgewählt unter Glycin, Alanin, Valin, Leucin und Isoleucin, bevorzugt Alanin, und
- A₆ eine Aminosäure darstellt, ausgewählt unter Glycin, Alanin, Valin, Leucin und Isoleucin, bevorzugt Glycin.

8. Synthetische Verbindung gemäß Anspruch 7, in welcher die Kohlenwasserstoffkette von peptidischer Art eine Sequenz umfasst, ausgewählt in der Gruppe, gebildet durch die Sequenzen:
-KGPGRAKG-
-KGFGRAKG-
-KPGGRAKG- und
-KFGGRAKG-.

9. Synthetische Verbindung gemäß irgendeinem der vorhergehenden Ansprüche, in welcher jedes Epitop wenigstens eine Sequenz umfasst, die in der Gruppe ausgewählt ist, gebildet durch die Sequenzen:
-TEPH,
-ALLGAR und
-FAEL.

10. Synthetische Verbindung gemäß irgendeinem der vorhergehenden Ansprüche, in welcher das Trägermolekül aus der Gruppe ausgewählt ist, die gebildet ist von: und Ac-KGPGRAKGPGSA-NH₂.

11. Verwendung einer synthetischen Verbindung gemäß einem der Ansprüche 1 bis 10 als Standard oder Nachweis für Immundosierungen von Troponin I.

12. Zusammensetzung, enthaltend wenigstens eine Verbindung gemäß irgendeinem der Ansprüche 1 bis 10 in Lösung in Wasser, im Plasma oder in der Pufferlösung.

13. Zusammensetzung gemäß Anspruch 12, **dadurch gekennzeichnet, daß** die Pufferlösung unter Phosphat-, Succinat- und Tris-HCl-Pufferlösungen ausgewählt ist.

14. Zusammensetzung gemäß Anspruch 13, in welcher die Pufferlösung in der Gruppe ausgewählt ist, die durch folgende Lösungen gebildet wird:
- Succinat-Pufferlösung (pH = 5-6), enthaltend ®Kathon, Regilait und EDTA,
- Succinat-Pufferlösung (pH = 5-6), enthaltend ®Kathon, Règilait, Casein und EDTA,
- Succinat-Pufferlösung (pH = 5-6), enthaltend ®Kathon und BSA,
- Phosphat-Pufferlösung (KH₂PO₄/K₂HPO₄ pH 6, 5-7, 5), enthaltend ®Kathon, Regilait und EDTA,
- Phosphat-Pufferlösung (KH₂PO₄/K₂HPO₄ 0,1 M pH 6,5-7,5), enthaltend ®Kathon, Casein und EDTA,
- Phosphat-Pufferlösung (KH₂PO₄/K₂HPO₄ 0,1 M pH 6,5-7,5), enthaltend ®Kathon und BSA.

15. Verfahren zur Immundosierung, welches als Standard oder Nachweis wenigstens eine synthetische Verbindung gemäß irgendeinem der Ansprüche 1 bis 10 verwendet.

16. Verfahren gemäß Anspruch 15, bei welchem die Immundosierung von Sandwich-Typ ist.

17. Zusammenstellung für Immundosierung, umfassend:
- wenigstens eine synthetische Verbindung gemäß irgendeinem der Ansprüche 1 bis 10 oder eine Zusammensetzung gemäß irgendeinem der Ansprüche 12 bis 14,
- wenigstens einen Reaktionsteilnehmer, der bei der Durchführung einer Immnundosierung üblichweise eingesetzt wird.


### SEQUENCE LISTING

<110> Bio-Rad Pasteur
<120> Composés synthétiques portant deux épitopes pour immunodosages
<130> BET 01/1239
<140> EP 00958689.2-1223
   <141> 2000-08-14
<160> 12
<170> PatentIn version 3.3
<210> 1
   <211> 8
   <212> PRT
   <213> Artificial
<220>
   <223> carrier molecule
<400> 1
   Lys Gly Pro Gly Arg Ala Lys Gly
   1               5
<210> 2
   <211> 8
   <212> PRT
   <213> Artificial
<220>
   <223> carrier molecule
<400> 2
   Lys Gly Phe Gly Arg Ala Lys Gly
   1 5
<210> 3
   <211> 8
   <212> PRT
   <213> Artificial
<220>
   <223> carrier molecule
<400> 3
   Lys Pro Gly Gly Arg Ala Lys Gly
   1 5
<210> 4
   <211> 8
   <212> PRT
   <213> Artificial
<220>
   <223> carrier molecule
<400> 4
   Lys Phe Gly Gly Arg Ala Lys Gly
   1 5
<210> 5
   <211> 4
   <212> PRT
   <213> Artificial
<220>
   <223> troponin I epitope
<400> 5
   Thr Glu Pro His
   1
<210> 6
   <211> 6
   <212> PRT
   <213> Artificial
<220>
   <223> troponin I epitope
<400> 6
   Ala Leu Leu Gly Ala Arg
   1 5
<210> 7
   <211> 4
   <212> PRT
   <213> Artificial
<220>
   <223> troponin I epitope
<400> 7
   Phe Ala Glu Leu
   1
<210> 8
   <211> 12
   <212> PRT
   <213> Artificial
<220>
   <223> carrier molecule
<220>
   <221> MISC_FEATURE
   <222> (1)..(12)
   <223> cyclic peptide
<400> 8
   Lys Gly Pro Gly Arg Ala Lys Gly Pro Gly Ser Ala
   1 5 10
<210> 9
   <211> 35
   <212> PRT
   <213> Artificial
<220>
   <223> peptide TRP 1116 Ac
<220>
   <221> MOD_RES
   <222> (1)..(1)
   <223> ACETYLATION
<220>
   <221> MISC_FEATURE
   <222> (17)..(17)
   <223> Xaa = Hx
<220>
   <221> MOD_RES
   <222> (35)..(35)
   <223> AMIDATION
<400> 9
   Gly Ser Ser Asn Tyr Arg Ala Tyr Ala Thr Glu Pro His Ala Lys Lys
   1               5                   10                  15
   Xaa Pro Leu Glu Leu Ala Gly Leu Gly Phe Ala Glu Leu Gln Asp Leu
               20                  25                  30
   Cys Arg Gln
           35
<210> 10
   <211> 12
   <212> PRT
   <213> Artificial
<220>
   <223> biepitopic compound
<220>
   <221> MISC_FEATURE
   <222> (1)..(12)
   <223> cyclic peptide
<220>
   <221> SITE
   <222> (1)..(1)
   <223> position of the side arm 1116 Ac
<220>
   <221> SITE
   <222> (7)..(7)
   <223> position of the side arm 1116 Ac
<400> 10
   Lys Gly Pro Gly Arg Ala Lys Gly Pro Gly Ser Ala
   1               5                   10
<210> 11
   <211> 12
   <212> PRT
   <213> Artificial
<220>
   <223> biepitopic compound
<220>
   <221> MOD_RES
   <222> (1)..(1)
   <223> ACETYLATION
<220>
   <221> SITE
   <222> (1)..(1)
   <223> position of the side arm 1116 Ac
<220>
   <221> SITE
   <222> (7)..(7)
   <223> position of the side arm 1116 Ac
<220>
   <221> MOD_RES
   <222> (12)..(12)
   <223> AMIDATION
<400> 11
   Lys Gly Pro Gly Arg Ala Lys Gly Pro Gly Ser Ala
   1               5                   10
<210> 12
   <211> 12
   <212> PRT
   <213> Artificial
<220>
   <223> biepitopic compound
<220>
   <221> MOD_RES
   <222> (1)..(1)
   <223> ACETYLATION
<220>
   <221> SITE
   <222> (1)..(1)
   <223> position of side arm 1116 Ac
<220>
   <221> SITE
   <222> (7)..(7)
   <223> position of side arm 1116 Ac
<220>
   <221> MOD_RES
   <222> (12)..(12)
   <223> AMIDATION
<400> 12
   Lys Gly Phe Gly Arg Ala Lys Gly Ser Gly Ser Ala
   1               5                   10